# EUROPEAN PATENT APPLICATION

(11) **EP 3 918 983 A1**
(43) Date of publication of application: **08.12.2021**
(21) Application number: 19912259.9
(22) Date of filing: 25.11.2019
(51) Int. Cl.: A61B 5/0245, A61B 5/11

(54) **INFORMATION PROCESSING DEVICE, INFORMATION PROCESSING METHOD, AND PROGRAM**

(30) Priority: 28.01.2019 JP 2019012053
(71) Applicant: Sony Group Corporation, 108-0075 Tokyo (JP)
(72) Inventor: SHIGYO, Maki, Tokyo 108-0075 (JP); KATSU, Masanori, Tokyo 108-0075 (JP)
(74) Representative: MFG Patentanwälte Meyer-Wildhagen Meggle-Freund Gerhard PartG mbB
(86) International application number: PCT/JP2019/045966
(87) International publication number: WO 2020/158129

(57) **Abstract**

An information processing device includes a control unit configured to perform control such that information for correcting vital sensing data acquired by a sensor in real time is presented.

## Description

### [Technical Field]

The present disclosure relates to an information processing device, an information processing method, and a program.

### [Background Art]

In the related art, devices that measure (determine) data related to living bodies (hereinafter appropriately referred to as vital sensing data) are known. For example, the following PTL 1 discloses a device that measures pulse waveforms accurately without putting a burden on a user.

### [Citation List]

### [Patent Literature]

[PTL 1]
JP 2013-121420A

### [Summary]

### [Technical Problem]

In general measurement devices that measure vital sensing data, there is a problem that vital sensing data cannot be corrected even when subjects (users) feel uneasy with measured values of the vital sensing data obtained by the devices.

The present disclosure has been devised in view of the above-described circumstances and an objective of the present disclosure is to provide an information processing device, an information processing method, and a program capable of correcting a measurement result of vital sensing data in real time.

### [Solution to Problem]

The present disclosure is, for example,
an information processing device including a control unit configured to perform control such that information for correcting time-series vital sensing data acquired by a sensor is presented.

The present disclosure is, for example,
an information processing method including performing control by a control unit such that information for correcting time-series vital sensing data acquired by a sensor is presented.

The present disclosure is, for example,
a program causing a computer to perform an information processing method of performing control by a control unit such that information for correcting time-series vital sensing data acquired by a sensor is presented.

### [Brief Description of Drawings]

[Fig. 1]

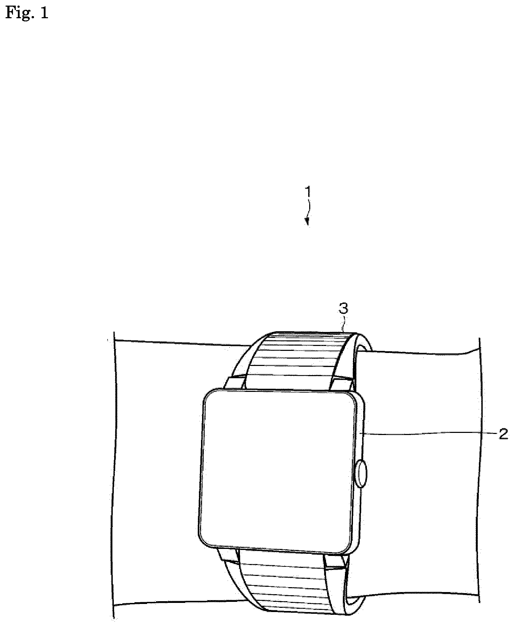

   Fig. 1 is a diagram illustrating an example of an exterior of a wearable device according to an embodiment.
[Fig. 2]

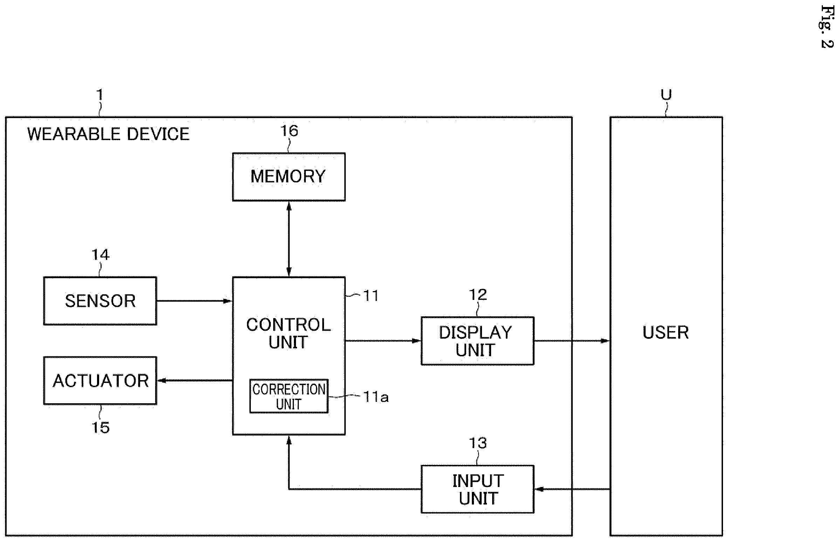

   Fig. 2 is a diagram illustrating an example of an internal configuration of the wearable device according to the embodiment.
[Fig. 3]

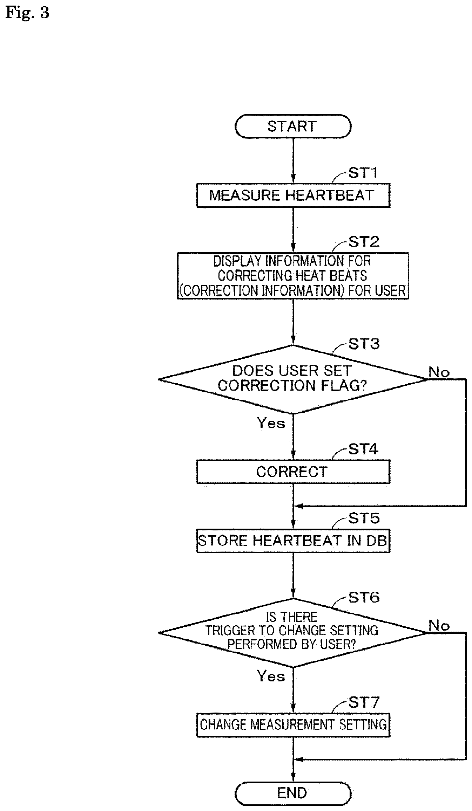

   Fig. 3 is a flowchart illustrating a flow of an overall process performed by the wearable device according to the embodiment.
[Fig. 4]

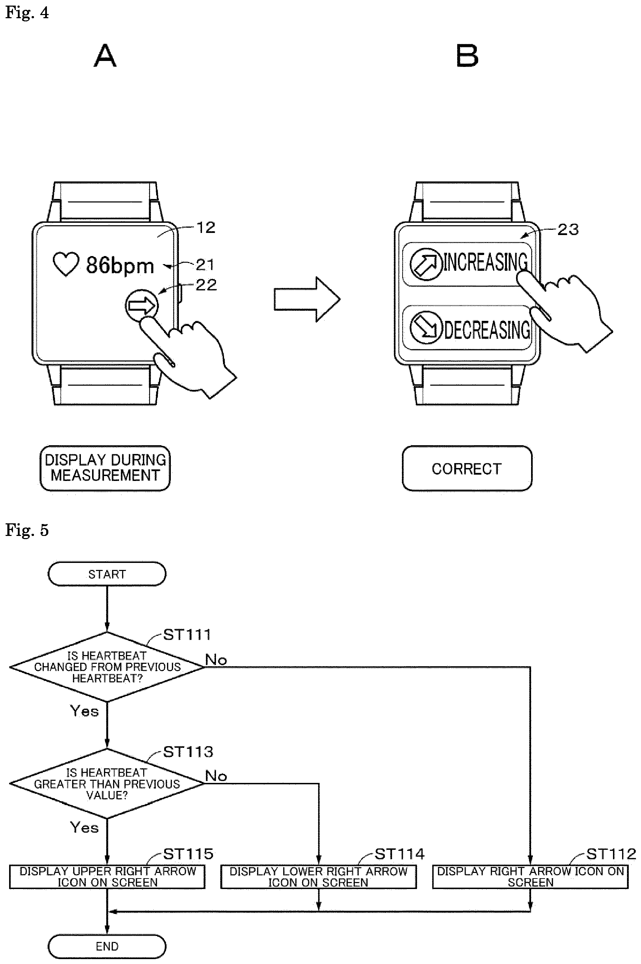

   Fig. 4A is a diagram illustrating an example of a heart rate display or the like in a first example and Fig. 4B is a diagram illustrating correction information in the first example.
[Fig. 5]
   Fig. 5 is a flowchart illustrating a flow of a process of displaying a heart rate change display in the first example.
[Fig. 6]

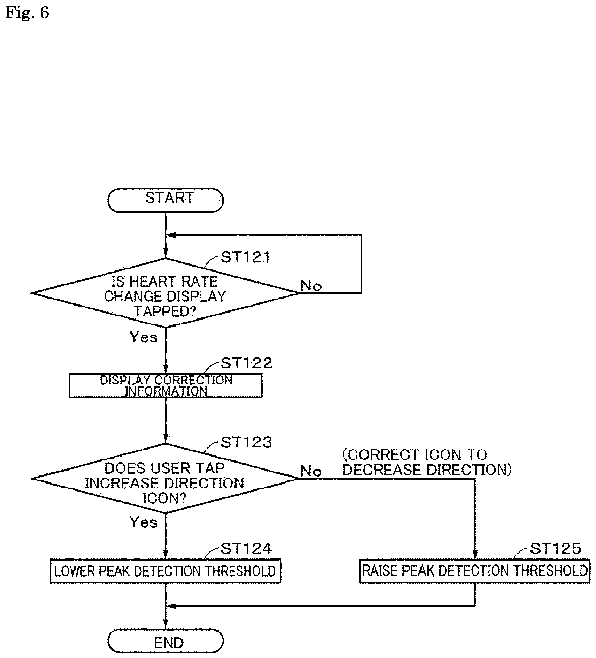

   Fig. 6 is a flowchart illustrating a flow of a process performed in response to an operation performed on the correction information in the first example.
[Fig. 7]

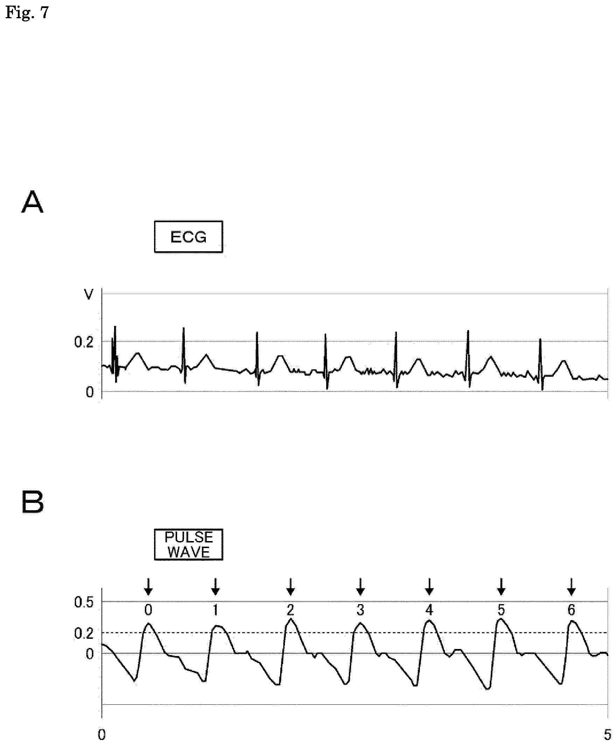

   Figs. 7A and 7B are diagrams illustrating an example of a method of detecting a heartbeat.
[Fig. 8]

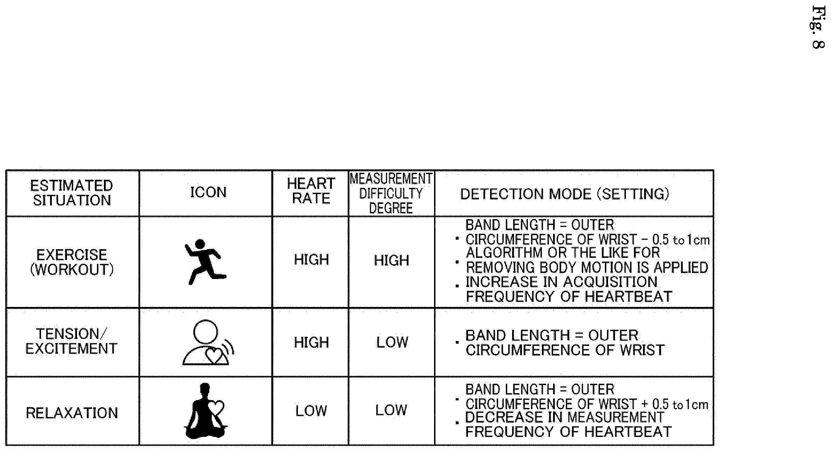

   Fig. 8 is a diagram illustrating an example of an estimated situation in a second example.
[Fig. 9]

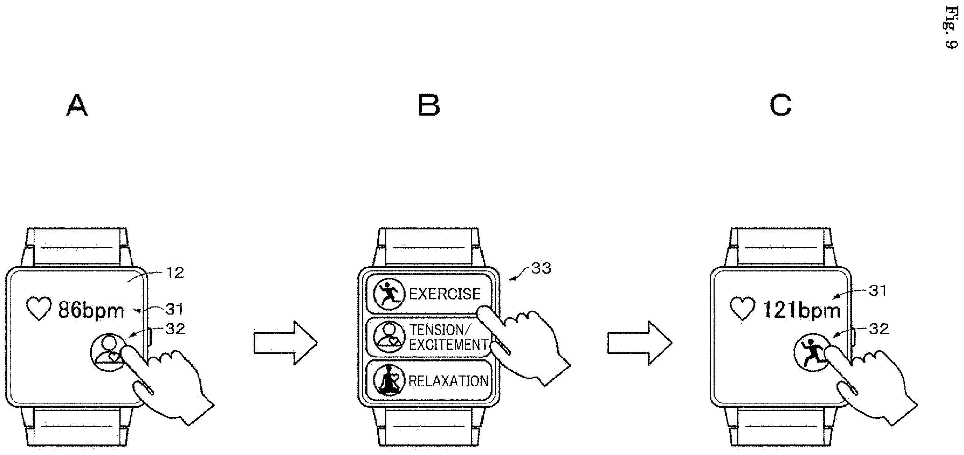

   Figs. 9A to 9C are diagrams schematically illustrating a process in the second example.
[Fig. 10]

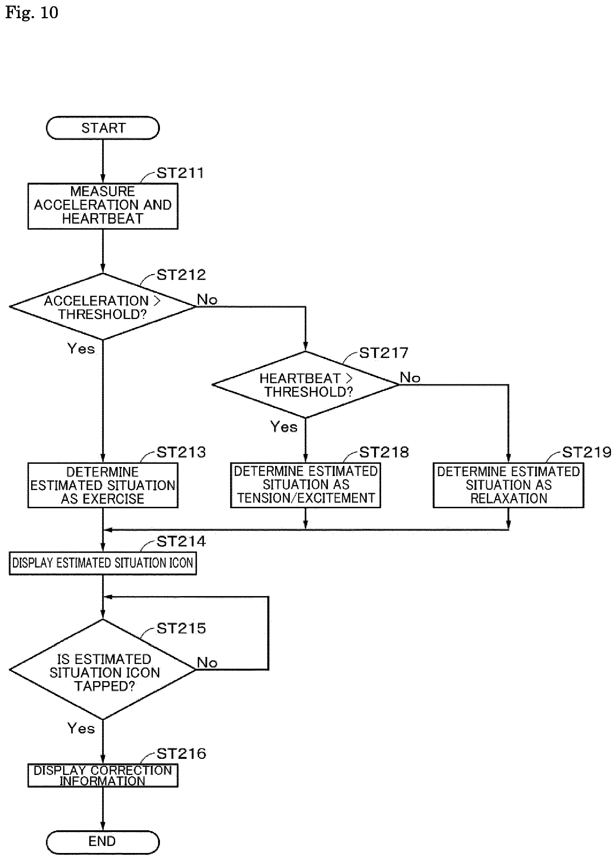

   Fig. 10 is a flowchart illustrating a flow of a process of displaying an estimated situation icon and correction information in the second example.
[Fig. 11]

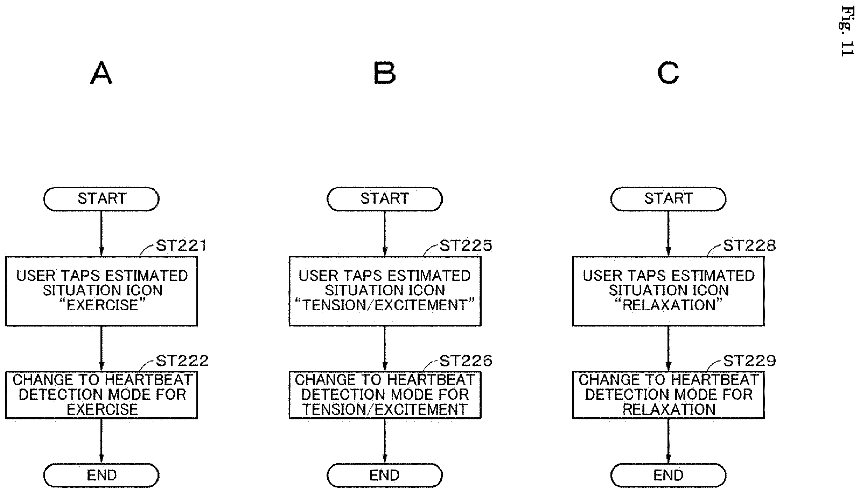

   Figs. 11A to 11C are flowcharts illustrating flows of processes performed in response to operations performed on the correction information in the second example.
[Fig. 12]

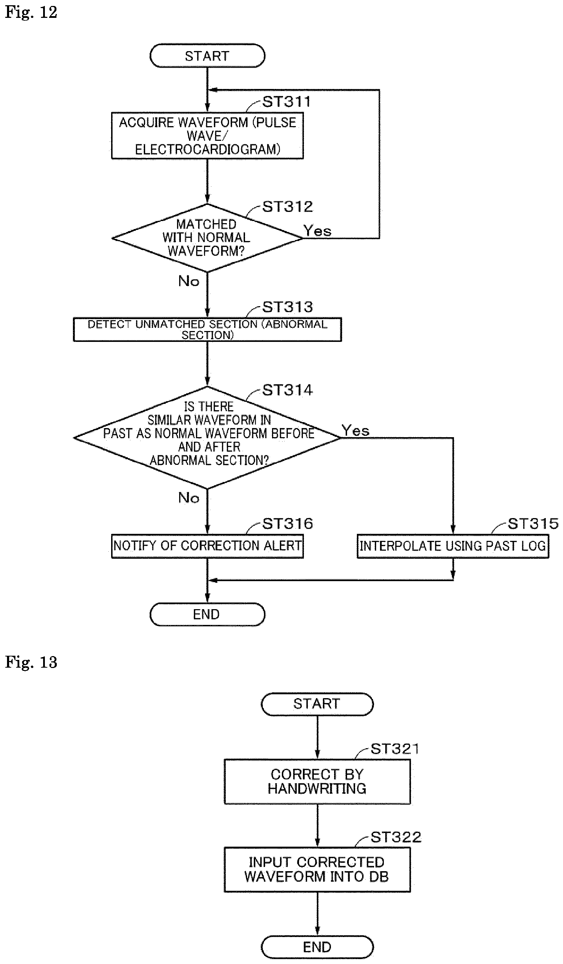

   Fig. 12 is a flowchart illustrating a flow of a process of presenting a correction alert to a user in a third example.
[Fig. 13]
   Fig. 13 is a flowchart illustrating a flow of a process performed in response to an operation performed on a correction alert in the third example.
[Fig. 14]

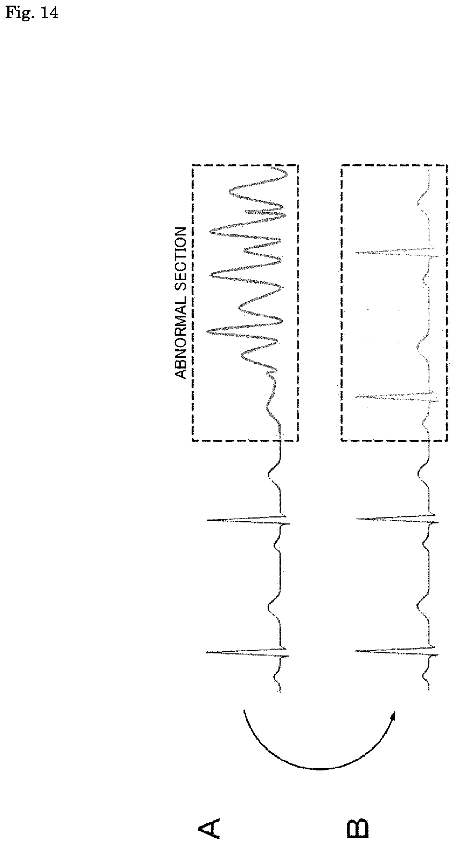

   Figs. 14A and 14B are diagrams schematically illustrating a form of correction of a user waveform in an abnormal section.
[Fig. 15]

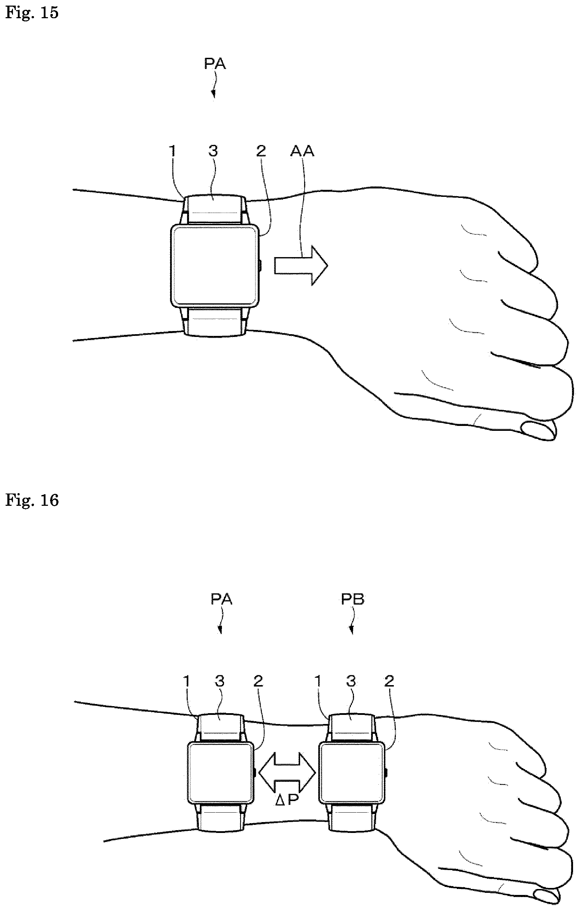

   Fig. 15 is a diagram illustrating an example of an appropriate measurement position of a wearable device according to an embodiment.
[Fig. 16]
   Fig. 16 is a diagram schematically illustrating a change in a measurement position of the wearable device according to the embodiment.
[Fig. 17]

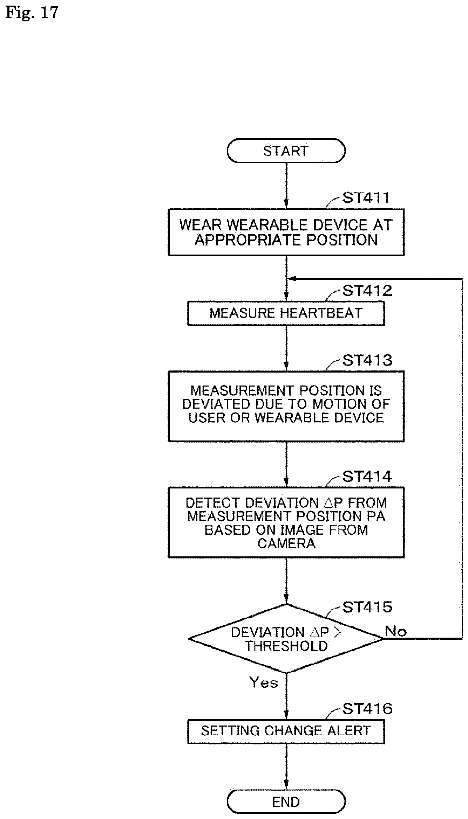

   Fig. 17 is a flowchart illustrating a flow of a process of presenting a setting change alert to a user U in a fourth example.
[Fig. 18]

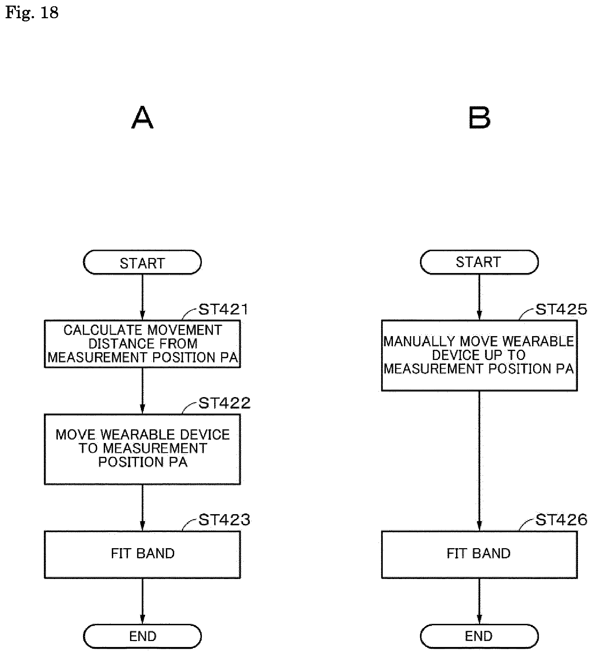

   Figs. 18A and 18B are flowcharts illustrating flows of processes performed in response to an operation performed on the setting change alert in the fourth example.
[Fig. 19]

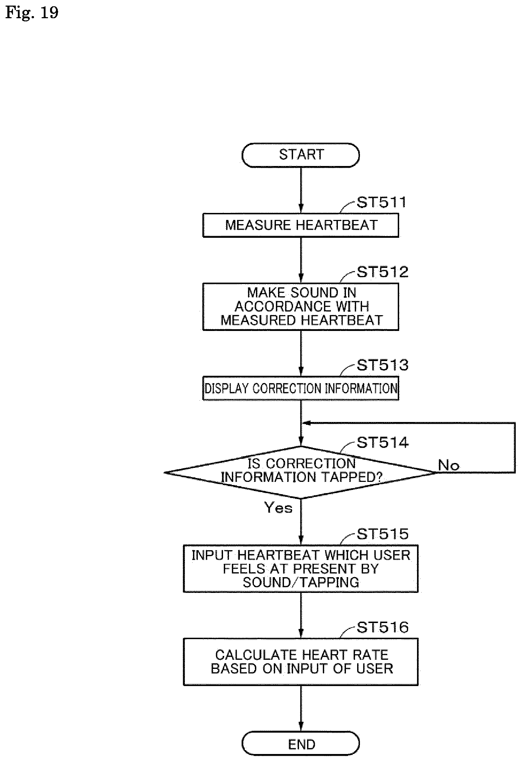

   Fig. 19 is a flowchart illustrating a flow of a process performed in response to an operation performed on correction information after the correction information is presented in a fifth example.
[Fig. 20]

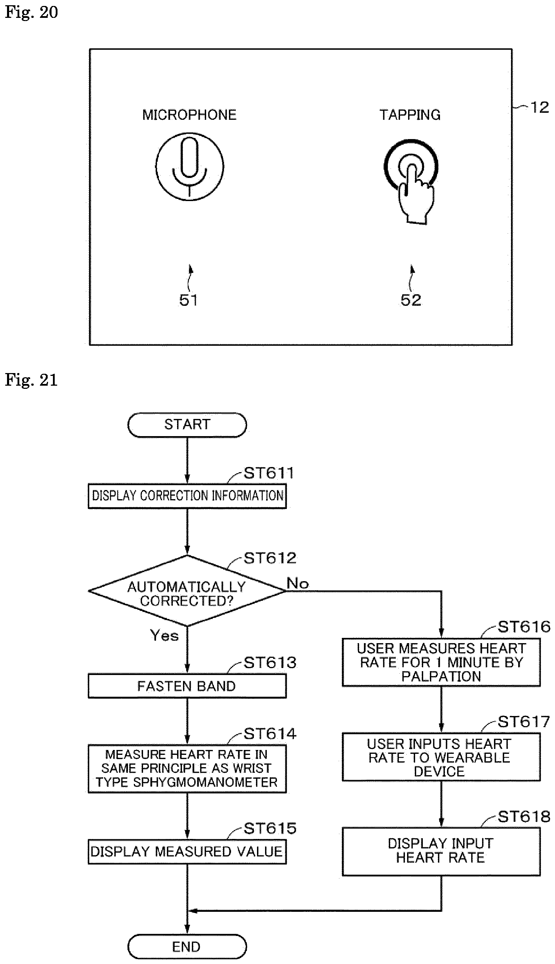

   Fig. 20 is a diagram illustrating an example of the correction information in the fifth example.
[Fig. 21]
   Fig. 21 is a flowchart illustrating a flow of a process performed in response to an operation performed on correction information after the correction information is presented in a sixth example.
[Fig. 22]

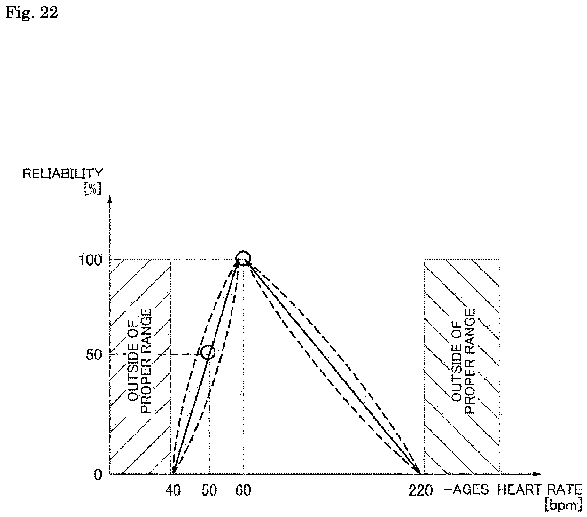

   Fig. 22 is a diagram illustrating a modified example.

### [Description of Embodiments]

Hereinafter, embodiments and the like of the present disclosure will be described with reference to the drawings. The description will be made in the following order.

### <Embodiment>

### <Modified examples>

The embodiments described below are preferred specific examples of the present disclosure, and the content of the present disclosure is not limited to the embodiments.

### <Embodiment>

### [Wearable device]

### (Example of exterior of wearable device)

An embodiment of the present disclosure will be described. In the embodiment, a wearable device will be described as an example of an information processing device. Fig. 1 is a diagram illustrating an example of an exterior of a wearable device (a wearable device 1) according to the embodiment. The wearable device 1 is, for example, a wristband type device and can measure vital sensing data of a user wearing the wearable device 1 on his or her wrist.

The wearable device 1 includes a body unit 2 and a ring-shaped band unit 3 connected to the body unit 2. A band length of the band unit 3 can be adjusted by at least one manual or automated operation.

### (Example of internal configuration of wearable device)

Fig. 2 is a diagram illustrating an example of an internal configuration of the wearable device 1. The wearable device 1 includes, for example, a control unit 11, a display unit 12 provided in the body unit 2, an input unit 13, a sensor 14, an actuator 15, and a memory 16. An input operation is performed on the wearable device 1 through the input unit 13 by a user U wearing the wearable device 1. Various kinds of information are presented to the user U in accordance with an appropriate method such as display, sound, or vibration by the wearable device 1.

The control unit 11 is configured from, for example, a central processing unit (CPU) and generally controls each unit of the wearable device 1. The control unit 11 according to the embodiment performs control such that information for correcting vital sensing data in a time series acquired by the sensor 14 (time-series vital sensing data) (hereinafter appropriately referred to as correction information) is presented. The correction information is, for example, information for correcting the time-series vital sensing data in real time. The control unit 11 includes a correction unit 11a that corrects vital sensing data in response to an input operation by the user U. As will be described in detail below, an example of a correction process performed by the correction unit 11a is at least one of a process of correcting the vistal data sensing data and a process of changing the setting of the wearable device 1 to acquire the vital sensing data and correcting a measurement result based on vital sensing data measured after the change.

The display unit 12 is a display formed of a liquid crystal display (LCD), electroluminescence (EL), or the like. Various kinds of information such as time are displayed on the display unit 12. The correction information is displayed on the display unit 12.

The input unit 13 is a generic unit with a configuration for receiving an input operation performed by the user U. Specific examples of the input unit 13 include a button, a touch switch, and a microphone.

The sensor 14 is a generic unit of a sensor measuring time-series vital sensing data of the user U, and an acceleration sensor, an image sensor, and the like that detect a motion of the user U. The types, number, arrangement positions, and the like of sensors configured as the sensor 14 can be appropriately changed in accordance with content of a process to be described below. In the embodiment, a heartbeat (which is data related to a heartbeat and includes a heart rate (a pulse rate), a waveform of a heartbeat or the like) will be described as an example of time-series vital sensing data.

The actuator 15 has, for example, a hardware configuration for changing a band length of the band unit 3 or a position of the wearable device 1. The actuator 15 operates under the control of the control unit 11.

The memory 16 is a generic unit of a ROM that stores a program executed by the control unit 11, a RAM that is used as a work memory when a program is executed, a memory that is used as a storage area of various kinds of data, and the like. As the memory 16, a magnetic storage device such as a hard disk drive (HDD), a semiconductor storage device, an optical storage device, a magneto-optical storage device, or the like can be applied.

Although an example of the internal configuration of the wearable device 1 has been described, the wearable device 1 may have a configuration different from the above-described configuration. For example, the wearable device 1 may include a communication unit that performs communication or a speaker that reproduces a sound. The communication performed by the communication unit may be wired communication or wireless communication. Examples of the wireless communication include a local area network (LAN), Bluetooth (registered trademark), Wi-Fi (registered trademark), and wireless USB (WUSB).

### [Process performed by wearable device]

### (Overall process)

Next, a process performed by the wearable device 1 will be described. First, a flow of an overall process performed by the wearable device 1 will be described with reference to the flowchart of Fig. 3. Specific content of an individual process will be described later. The process in the flowchart to be described below is performed under the control of the control unit 11 unless otherwise mentioned. The same applies to processes in other flowcharts.

In step ST1, the sensor 14 measures a heartbeat. Then, the process proceeds to step ST2.

In step ST2, correction information which is information for correcting the heartbeat in real time is displayed to the user U. Then, the process proceeds to step ST3.

In step ST3, it is determined whether the user U sets a correction flag. Here, the setting of the correction flag is an input operation of correcting the measured heartbeat. When the correction flag is set, the process proceeds to step ST4. When the correction flag is not set, the process proceeds to step ST5.

In step ST4, the heartbeat is corrected. Then, the process proceeds to step ST5.

In step ST5, at least one of the heartbeat measured by the sensor 14 and a heartbeat corrected by the user U is stored in a database (DB). The database DB may be a memory 16 of the wearable device 1, may be a database on a cloud, or may be a memory of a device different from the wearable device 1.

Then, the process proceeds to step ST6. In step ST6, it is determined whether a trigger to change the setting for measuring the heartbeat is performed by the user. When there is no trigger, the process ends. When there is a trigger to change the setting for measuring the heartbeat, the process proceeds to step ST7. In step ST7, the setting for measuring the heartbeat is changed and the time-series vital sensing data is measured based on the changed setting. Then, the process ends.

### (First example)

Next, a specific example of each of the above-described processes will be described. First, a first example will be described. In the first example, a tendency to change the heart rate is displayed on the display unit 12. When the displayed tendency to change the heart rate does not match a tendency to change a sensory (subjective) tendency to change a heartbeat, the user U the heart rate using the correction information displayed on the display unit 12.

Fig. 4A is a diagram illustrating a display example (a display example during measurement) of a heart rate or the like in the first example. As illustrated in Fig. 4A, the heart rate measured by the sensor 14 is displayed as heart rate display 21 on the display unit 12. In the example illustrated in Fig. 4A, "86 bpm" is displayed as the heart rate display 21. The value of the heart rate display 21 is changed in real time. For example, in the lower right of the heart rate display 21, a heart rate change display 22 indicating a change in the heart rate is displayed. When the heart rate is not changed, as illustrated in Fig. 4A, a right arrow is displayed as the heart rate change display 22. When the heart rate tends to increase, an upper right arrow is displayed. When the heart rate tends to decrease, a lower right arrow is displayed. The display representing the tendency to show the heart rate may not be an arrow, but may be a sign other than text or an arrow.

In the heart rate change display 22, the user U can easily recognize a difference from the sensory heart rate. For example, when the right arrow which is a display representing that the heart rate is hardly changed or the lower right arrow indicating a tendency to decrease the heart rate is displayed as the heart rate change display 22 despite the fact that the user U is exercising (which may be light-load exercise), the user U may feel uneasy. In the embodiment, in consideration of this case, the user U is allowed to be able to correct data related to the measured heartbeat in real time based on a bodily sensation.

For example, the user U who feels uneasy with a present measurement result taps the heart rate change display 22. In response to the tap operation, display content of the display unit 12 transitions from a screen illustrated in Fig. 4A to a screen illustrated in Fig. 4B and correction information 23 is displayed on the display unit 12. In this example, the correction information 23 is, for example, a heart rate change display corresponding to a change tendency different from the heart rate change display 22 displayed during the measurement. The correction information 23 is information indicating a tendency to change time-series vital sensing data and includes at least two of an increase, a decrease, and a non-change. As illustrated in Fig. 4A, in this example, the right arrow is displayed during measurement and the user U feels uneasy with that, and thus "(upper right arrow), increasing" and "(lower right arrow), decreasing" are displayed as the correction information 23. When the user U feels the increase in the heart rate as his or her own bodily sensation, the user U taps "(upper right arrow), increasing" in the correction information 23. When the user U feels the decrease in the heart rate as his or her own bodily sensation, the user U taps "(lower right arrow), decreasing" in the correction information 23. In this example, the setting for acquiring the heartbeat is changed in response to the tap operation.

A flow of a process performed in the first example will be described with reference to the flowcharts of Figs. 5 and 6. Fig. 5 is a flowchart illustrating a flow of a process of displaying the heart rate change display 22 and is a specific flowchart illustrating content of the process of step ST1 mainly described above.

After the process starts, in step ST111, it is determined whether the measured heart rate is changed from the previous heart rate. Here, when the heart rate is not changed from the previous heart rate, the process proceeds to step ST112. In step ST112, the "right arrow" is displayed as the heart rate change display 22 on the display unit 12. Then, the process ends. When the measured heart rate is changed from the previous heart rate in the determination process of step ST111, the process proceeds to step ST113.

In step ST113, it is determined whether the heart rate is greater than the previous value. Here, when the heart rate is not greater than the previous value, in other words, when the heart rate is less than the previous value, the process proceeds to step ST114. In step ST114, since the heart rate tends to decrease, the lower right arrow is displayed as the heart rate change display 22. Then, the process ends. When the heart rate is greater than the previous value in the determination process of step ST113, the process proceeds to step ST115. In step ST115, since the heart rate tends to increase, the upper right arrow is displayed as the heart rate change display 22. Then, the process ends.

For example, when the heart rate is changed within 3 bpm, it is determined that the heart rate is not changed. In this way, a determination reference for a non-change in the heart rate, a tendency to increase the heart rate, a tendency to decrease the heart rate, and the like can be appropriately set.

Fig. 6 is a flowchart illustrating a flow of a process performed in response to an operation performed on the correction information when the correction information is displayed. In this example, when the correction information is operated, the setting for measuring the heartbeat is changed. Accordingly, the flowchart illustrated in Fig. 6 is a specific flowchart illustrating the content of steps ST2, ST6, and ST7 of the overall process.

After the process starts, in step ST121, it is determined whether the heart rate change display 22 is tapped. When the heart rate change display 22 is not tapped, the process returns to step ST121 and the determination process of step ST121 is repeated. When the heart rate change display 22 is tapped, the process proceeds to step ST122.

In step ST122, the correction information 23 (see Fig. 4B) is displayed on the display unit 12. Then, the process proceeds to step ST123.

In step ST123, content of the operation performed on the correction information by the user U is determined. For example, it is determined whether the user U taps an increase direction icon (the upper right arrow). When the upper right arrow is tapped, the process proceeds to step ST124.

In step ST124, the control unit 11, specifically, the correction unit 11a, performs control such that a threshold for detecting a heartbeat is lowered. Then, the process ends.

When the upper right arrow is not tapped and a decrease direction icon (the lower right arrow) is tapped in the process of step ST123, the process proceeds to step ST125. In step ST125, the correction unit 11a performs control such that the threshold for detecting a heartbeat is raised. Then, the process ends.

As illustrated in Fig. 7A, a threshold (0.2 V in the example illustrated in Fig. 7A) for detecting a heartbeat is set in a waveform of an electrocardiogram (ECG). As a method of detecting a heartbeat, there is a method of using a pulse wave. In this method, as illustrated in Fig. 7B, a threshold (0.2 V in the example illustrated in Fig. 7B) is set in a pulse wave. A portion in which a voltage equal to or greater than a threshold is detected is considered to be a heartbeat. The tapping of the upper right arrow of the correction information 23 means that the user U senses that the heart rate further increases. Accordingly, as described above, the threshold can be lowered in response to the operation of tapping the upper right arrow, and thus it is considered easy to detect the heart rate. On the other hand, the tapping of the lower right arrow of the correction information 23 means that the user U senses that the heart rate further decreases. Accordingly, as described above, the threshold is raised in response to an operation of tapping the lower right arrow, and thus it is considered difficult to detect the heart rate. The degree of change in the threshold can be appropriately changed. The threshold may be changed step by step in response to an operation of tapping the increase direction icon or the decrease direction icon a plurality of times. The heartbeat is measured based on the changed threshold and a measurement result is corrected using the measured heartbeat.

Although not illustrated, data such as the heart rate measured after the change in the setting is stored in the memory 16. The heart rate before the change in the setting may be stored or may be discarded. A boundary between before and after the change in the setting may be understandable and the change in the heart rate may be stored.

As the user U who can use the first example, a user who can recognize an increase or decrease in the heart rate by himself or herself is assumed. In the foregoing description, the threshold for detecting the heartbeat is changed in response to an operation performed on the correction information 23, but an algorithm for detecting a heartbeat may be changed or a setting (for example, a filter coefficient) of an electrical circuit for detecting a heartbeat may be changed.

### (Second example)

In a second example, the control unit 11 of the wearable device 1 estimates a situation of a user and displays an estimated situation icon based on an estimation result. The user U taps another estimated situation icon when the user U bodily feels uneasy with the displayed estimated situation icon. The setting for measuring a heartbeat is changed in response to this operation.

Fig. 8 is a diagram illustrating an example of an estimated situation in the second example. In this example, "Exercise,", "Tension/Excitement," and "Relaxation" are set as estimated situations. In each estimated situation, an icon corresponding to the estimated situation (an estimated situation icon), characteristics of a heart rate and the measurement difficulty degree, and a detection mode (settings for measuring the heart rates) are associated. The detection mode includes at least one of a hardware setting (for example, a band length) and a software setting.

In the estimated situation "Exercise," the heart rate increases and a body motion is involved, and thus the degree of difficulty in measurement of the heartbeat increases. Accordingly, as the detection mode corresponding to the estimated situation "Exercise," the band length is set to be shorter than the outer circumference of a wrist by about 0.5 cm to 1 cm for tightening. To reduce an influence of a body motion on the measurement, an algorithm or the like for removing a body motion is applied. Since the heart rate increases, an acquisition frequency of a heartbeat is considered to increase.

In the estimated situation "Tension/Excitement," the heart rate increases and a body motion decreases, and thus the degree of difficulty in measurement of the heartbeat decreases. Accordingly, as a detection mode corresponding to the estimated situation "Tension/Excitement," the band length is set to about the outer circumference of a wrist.

In the estimated situation "Relaxation," the heart rate decreases and a body motion decreases, and thus the degree of difficulty in measurement of the heartbeat decreases. Accordingly, as the detection mode corresponding to the estimated situation "Relaxation," the band length is set to be longer than the outer circumference of a wrist by about +0.5 cm to 1 cm for loosening. Since the heart rate decreases, an acquisition frequency of a heartbeat is considered to decrease.

A process in the second example will be described schematically with reference to Figs. 9A to 9C. Fig. 9A is a diagram illustrating a display example during measurement of a heartbeat. On the display unit 12, heart rate display 31 (86 bpm in the illustrated example) indicating a heart rate is displayed. On the display unit 12, an estimated situation icon 32 is displayed. Here, for example, when the user U is exercising, the heart rate does not increase, and the user U feels uneasy with content of the estimated situation icon 32, the user U determines that the estimated situation determined by the wearable device 1 is wrong and corrects the estimated situation.

For example, the user U taps a portion of the estimated situation icon 32. Then, display content of the display unit 12 transitions from content illustrated in Fig. 9A to content illustrated in Fig. 9B. As illustrated in Fig. 9B, all the three estimated situation icons are displayed as correction information 33 on the display unit 12. The user U taps, for example, the estimated situation icon of "the estimated situation "Exercise." Then, the setting for acquiring the heartbeat is switched to the detection mode corresponding to the estimated situation "Exercise." The heart rate is corrected in accordance with the heart rate (121 bpm in the illustrated example) measured in the switched detection mode and the corrected heart rate is displayed as the heart rate display 31. The estimated situation icon 32 is switched to an icon corresponding to the estimated situation "Exercise." In this way, the user U can change the estimated situation to match the own bodily sensation and displays the heart rate measured in the detection mode in accordance with the bodily sensation. By using the estimated situation icon which can be intuitively operated rather than directly correcting the heart rate, the user U who does not have expert knowledge can also change the heartbeat detection mode easily.

The band length may be changed, for example, in such a manner that he actuator 15 is driven under the control of the control unit 11, may be changed by filling the inside of the band length with a gas such as the air, or may be changed a known method.

A flow of a process performed in the second example will be described with reference to the flowcharts of Figs. 10 and 11. Fig. 10 is a flowchart illustrating a flow of a process of displaying an estimated situation icon and correction information and is a specific flowchart illustrating content of the process of steps ST1 and ST2 mainly described above.

After the process starts, in step ST211, the sensor 14 measures acceleration and a heartbeat of the user U. Then, the process proceeds to step ST212.

In step ST212, when the acceleration acquired by the sensor 14 is compared with a threshold and it is determined whether there is a body motion of the user U. When the acceleration is greater than the threshold, the process proceeds to step ST213. Since the acceleration is greater than the threshold, it is determined that there is the body motion of the user U. Accordingly, in step ST213, the estimated situation is determined to be "Exercise." Then, the process proceeds to step ST214.

In step ST214, the estimated situation icon 32 corresponding to the determined estimated situation is displayed (see Fig. 9A). In this example, the heart rate display 31 is also displayed along with the estimated situation icon 32. Then, the process proceeds to step ST215.

In step ST215, it is determined whether the estimated situation icon 32 is tapped. When the estimated situation icon 32 is not tapped, the process returns to step ST215. When the estimated situation icon 32 is tapped, the process proceeds to step ST216.

In step ST216, since the estimated situation icon 32 is tapped, the correction information 33 is displayed on the display unit 12 (see Fig. 9B). Then, the process ends.

When the acceleration is equal to or less than the threshold in the determination process of step ST212, the process proceeds to step ST217. In step ST217, the heartbeat measured by the sensor 14 is compared with a heartbeat threshold. When the heartbeat is greater than the threshold as a comparison result, the process proceeds to step ST218.

In step ST218, since the body motion of the user is small and the heartbeat is high, the estimated situation is determined to be "Tension/Excitement." Then, the process proceeds to step ST214. Since the process after step ST214 has already been described, repeated description will be omitted.

When the heartbeat is equal to or less than the threshold in the determination process of step ST217, the process proceeds to step ST219. In step ST219, since the body motion of the user is small and the heartbeat is low, the estimated situation is determined to be "Relaxation." Then, the process proceeds to step ST214. Since the process after step ST214 has already been described, repeated description will be omitted.

Figs. 11A to 11C are flowcharts illustrating flows of processes performed in response to operations performed on the correction information (correction information 33). In this example, when the correction information is operated, the setting for measurement the heartbeat (the detection mode) is changed. Accordingly, the processes of the flowcharts illustrated in Figs. 11A to 11C are specific processes of the content of the process of steps ST6 and ST7 mainly described above.

After the process in the flowchart illustrated in Fig. 11A starts, in step ST221, the estimated situation icon of "Exercise" in the correction information 33 is tapped. Then, the process proceeds to step ST222.

In step ST222, the correction unit 11a changes the detection mode for detecting the heartbeat to the detection mode corresponding to the estimated situation "Exercise" in response to the operation of step ST221. The heartbeat is measured in accordance with the changed detection mode. Then, the process ends.

After the process in the flowchart illustrated in Fig. 11B starts, in step ST225, the estimated situation icon of "Tension/Excitement" in the correction information 33 is tapped. Then, the process proceeds to step ST226.

In step ST226, the correction unit 11a changes the detection mode for detecting the heartbeat to the detection mode corresponding to the estimated situation "Tension/Excitement" in response to the operation of step ST225. The heartbeat is measured in accordance with the changed detection mode. Then, the process ends.

After the process in the flowchart illustrated in Fig. 11C starts, in step ST228, the estimated situation icon of "Relaxation" in the correction information 33 is tapped. Then, the process proceeds to step ST229.

In step ST229, the correction unit 11a changes the detection mode for detecting the heartbeat to the detection mode corresponding to the estimated situation "Relaxation" in response to the operation of step ST226. The heartbeat is measured in accordance with the changed detection mode. Then, the process ends.

As the user U who can use the second example, a user who has some knowledge that a heart rate becomes high during exercise or in a sympathetic nerve predominant state and a heart rate becomes low in a relaxed state is assumed. The estimated situations are not limited to the above-described three estimated situations and other estimated situations may be set. The user U may customize icons of the estimated situations or content of the detection modes.

### (Third example)

First, a third example will be described. The third example is an example in which the user U can directly correct data related to a heartbeat when a deficiency or the like occurs due to a certain reason in data (a pulse wave or electrocardiogram) related to the heartbeat acquired by the sensor 14. That is, the third example is an example which time-series vital sensing data can be corrected. The correction is not limited to an operation by the user U. The correction may be performed automatically or manual or automatic correction may be allowed to be selected by the user U.

A flow of a process performed in the third example will be described with reference to the flowchart of Fig. 12. Fig. 12 is an example in which a correction alert which is an example of the correction information is presented to the user U and is a specific flowchart illustrating content of the process of steps ST1 and ST2 mainly described above.

In step ST311, a waveform of a heartbeat (pulse wave/electrocardiogram) by the sensor 14 is acquired chronologically and the acquired wave (hereinafter appropriately referred to as a user waveform) is displayed on the display unit 12. Then, the process proceeds to step ST312.

In step ST312, a process of matching a user waveform with a normal waveform (which is an example of a predetermined pattern) generally considered to be normal is performed. As the matching process, a known process such as a process of determining correlation can be applied. When a difference between the user waveform and the normal waveform is within a given range as a matching result, the user waveform and the normal waveform are determined to be matched and the process returns to step ST311. When the difference between the user waveform and the normal waveform is equal to or greater than the given range as a matching result, the process proceeds to step ST313. In this example, the case in which the difference between the user waveform and the normal waveform is equal to or greater than the given range is assumed to be a case in which deficiency or apparent abnormality occurs in a part of the user waveform due to a certain reason such as a shock to the wearable device 1 rather than body abnormality.

In step ST313, a section in which the user waveform and the normal waveform are not matched (hereinafter appropriately referred to as an abnormal section) is detected. Then, the process proceeds to step ST314.

In step ST314, it is determined whether there is a similar wave in the past in the memory 16 as a normal waveform before and after the abnormal section. When there is the similar wave in the past in the memory 16 as a normal waveform before and after the abnormal section, the process proceeds to step ST315.

In step ST315, a waveform in the abnormal section is interpolated in accordance with the past normal waveform using a log (a past waveform) stored in the memory 16. Then, the process ends.

When there is no similar wave in the past in the memory 16 as a normal waveform before and after the abnormal section in the determination process of step ST314, the process proceeds to step ST316. In step ST316, a correction alert which is an example of the correction information is presented to the user U.

The correction alert is information that notifies the user U that there is a section in which normal time-series vital sensing data is not acquired and is, for example, highlight display of the abnormal section or a warning sound, vibration, or the like indicating that the abnormal section is detected. Then, the process ends.

Fig. 13 is a flowchart illustrating a flow of a process performed in response to an operation on the correction information (a correction alert). In this example, when the correction information is operated, the waveform of the heartbeat is corrected. Accordingly, the process of the flowcharts illustrated in Fig. 13 is a specific process of the content of the process of steps ST3 and ST4 mainly described above.

After the process starts, in step ST321, for example, a handwritten user waveform is corrected. As illustrated in Fig. 14A, the waveform in an abnormal section is corrected in accordance with an appropriate method such as a touching operation performed with a stylus pen, a mouse, a finger, as schematically illustrated in Fig. 14B. Then, the process proceeds to step ST322.

In step ST322, the corrected user waveform is stored in the memory 16 or a database on a cloud. Then, the process ends.

As the user U who can use the third example, a user who has some knowledge about a waveform or meaning of a pulse wave/electrocardiogram is assumed. When the user of the wearable device 1 is a doctor, a nurse, or the like, the user can correct the waveform in real time despite a deficient portion in the user waveform.

The operation of correcting the user waveform may be performed with the wearable device 1 or may be performed with a device different from the wearable device 1. For example, the user waveform is transmitted to a personal computer, a tablet computer, or the like in real time and the user waveform is displayed on the personal computer or the like. Then, the correction operation may be performed on the user waveform displayed on the personal computer or the like. An input performed to correct the waveform of the time-series vital sensing data may be a sound input or the like.

The control unit 11 may perform machine learning using a portion of the corrected user waveform as training data. Then, a result of the machine learning may be applied in the process of step ST315 described above. Even when the user waveform becomes deficient by the same noise later by applying the result of the machine learning, the user waveform can be corrected automatically and appropriately. The above-described process of matching the user waveform with the normal waveform may be performed for each waveform or may be performed in units of a plurality of waveforms.

### (Fourth example)

First, a fourth example will be described. To appropriately acquire time-series vital sensing data, it is necessary to locate the wearable device 1, specifically, the sensor 14 acquiring the time-series vital sensing data, at an appropriate measurement position. The fourth example is an example in which the time-series vital sensing data is corrected by presenting the correction information and correcting the position of the sensor 14 to the appropriate measurement position in response to an operation on the correction information when the position of the sensor 14 is deviated from the appropriate measurement position in view of this necessity. That is, the fourth example is an example in which a hardware setting (a physical setting) for acquiring the time-series vital sensing data is changed in response to an operation on the correction information.

In this example, the appropriate measurement position of the wearable device 1 is input in advance. For example, as illustrated in Fig. 15, the appropriate measurement position (hereinafter appropriately referred to as a measurement position PA) of the wearable device 1 worn near a wrist is input. To input the measurement position, it is necessary to acquire positional information of the measurement position PA. In this example, an image sensor is provided on a lateral side (a surface parallel to an extension direction of the band unit 3) of the body unit 2 of the wearable device 1. Imaging is performed in a direction indicated by an arrow AA of Fig. 15 (the back side of a hand). A feature point (which is an example of information regarding a feature of a user) of an overhang of the back of the hand is set. A distance to the feature point is measured using a depth camera, a time of flight (ToF) sensor, or the like. The obtained distance to the feature point is set as positional information of the measurement position PA. The measurement position PA set in advance can also be changed later.

Based on information regarding the feature of the user, a setting related to the position of the sensor is changed. For example, as illustrated in Fig. 16, the measurement position of the wearable device 1 is assumed to be changed by ΔP from the measurement position PA to a measurement position PB as the wearable device 1 is used. When the change ΔP is equal to greater than a given value, it is determined that the wearable device 1 is at a position at which a heartbeat cannot accurately be measured and a setting change alert is presented as correction information to the user U.

A flow of a process performed in the fourth example will be described with reference to the flowchart of Fig. 17. Fig. 17 is a flowchart illustrating a flow of a process of presenting a setting change alert which is an example of correction information to the user U and is a specific flowchart illustrating content of the process of steps ST1 and ST2 mainly described above.

After the process starts, in step ST411, the wearable device 1 is worn at an appropriate position (the measurement position PA in this example). Then, the process proceeds to step ST412.

In step ST412, a heartbeat is measured by the sensor 14 of the wearable device 1 which is at the measurement position PA. Then, the process proceeds to step ST413.

In step ST413, the position of the wearable device 1 becomes deviated from the measurement position PA due to a motion of the user U or the wearable device 1. Then, the process proceeds to step ST414.

In step ST414, the deviation ΔP of the distance to the feature point before and after the change in the position is detected based on an image acquired by a camera of the wearable device 1. Then, the process proceeds to step ST415.

In step ST415, it is determined whether the deviation ΔP is greater than a predetermined threshold. When the deviation ΔP is equal to or less than the threshold, the process returns to step ST412. When the deviation ΔP is greater than the threshold, the process proceeds to step ST416.

In step ST416, a setting change alert which is an example of the correction information is presented to the user U. The setting change alert is presented to the user U by display, sound, vibration, or the like such as "The position of the wearable device is deviated from the appropriate position." Then, the process ends.

Figs. 18A and 18B are flowcharts illustrating flows of processes performed in response to an operation (for example, an operation of tapping a correction button or inputting a sound) on the correction information (the setting change alert). The processes of the flowcharts illustrated in Figs. 18A and 18B are mainly specific processes of step ST7 in all the processes. Fig. 18A is a flowchart illustrating a flow of a process of automatically correcting a measurement position of the wearable device 1 (the sensor 14) to the measurement position PA in response to an operation on the setting change alert.

In step ST421, a distance from a present position of the wearable device 1 to the measurement position PA is calculated. The calculated distance is a movement distance for correcting the measurement position of the wearable device 1 to the measurement position PA. Then, the process proceeds to step ST422.

In step ST422, the wearable device 1 is moved to the measurement position PA in response to a trigger of the setting change alert (a predetermined input). Then, the process proceeds to step ST423.

In step ST423, the band unit 3 is fastened and the wearable device 1 is re-mounted at the measurement position PA.

Specific examples of steps ST422 and ST423 will be described. For example, by making it possible to inject or discharge the air or a gas into the band unit 3, it is possible to change a band length (the degree of fastening) of the band unit 3. Discharging holes for air or gas are provided on both sides of the wearable device 1. In the wearable device 1 that has the foregoing configuration, the measurement position of the wearable device 1 is changed as follows. First, an amount of air inside the band unit 3 is adjusted to loosen the band unit 3. Then, the air is sequentially ejected from the sides of the wearable device 1 so that the wearable device 1 is moved in a direction oriented to the measurement position PA. The ejection of the air is repeated until the wearable device 1 arrives at the measurement position PA. In a stage at which the wearable device 1 arrives at the measurement position PA, the ejection of the air is stopped and the band unit 3 can be appropriately fastened by adjusting an amount of air inside the band unit 3. Since the wearable device 1 is moved to the measurement position PA, the sensor 14 can measure a heartbeat at an appropriate position, and thus the correction can be performed based on the heartbeat measured at the appropriate position.

The air may not be ejected and the wearable device 1 may be moved to the measurement position PA to be suitable for natural arm swing. For example, after the band unit 3 is loosened, the wearable device 1 is moved using a motion of the user U such as arm swing. Whether the wearable device 1 is at the measurement position PA is determined at an appropriate interval even during the movement of the wearable device 1. When the wearable device 1 is moved to the measurement position PA, the band unit 3 can be appropriately fastened and the wearable device 1 is corrected to the measurement position PA. By applying an acceleration sensor as a constituent element of the sensor 14 and allowing the acceleration sensor to detect that an arm is oriented downwards, the band unit 3 can be loosened, and thus the wearable device 1 may be moved.

A configuration in which a rail mechanism is provided and the rail mechanism is slidable in a state in which the wearable device 1 can measure a heartbeat may be adopted. The rail mechanism is worn on an arm (for example, a position from an elbow to the vicinity of a wrist) of the user U. When the wearable device 1 is deviated from the measurement position PA, control may be performed such that the wearable device 1 is moved on the rail mechanism to return to the measurement position PA.

Fig. 18B is a flowchart illustrating a flow of a process of manually correcting a measurement position of the wearable device 1 (specifically, the sensor 14) to the measurement position PA in response to an operation on the setting change alert.

After the process starts, in step ST425, the user U loosens the band unit 3 by himself or herself. Then, the user U manually moves the wearable device 1 up to the measurement position PA. The user U may be informed of the movement of the wearable device 1 up to the measurement position PA. Then, the process proceeds to step ST426.

In step ST426, the user U fastens the band unit 3 to correct the position of the wearable device 1 to the measurement position PA.

In the foregoing description, the example in which the positional deviation occurring in the arm direction in the wearable device 1 is corrected has been described, but a positional deviation occurring in a direction perpendicular to the arm direction may be corrected automatically or manually. The feature point may be information (for example, a branch point of a vein or the position of a sweat gland) obtained by imaging an arm side.

### (Fifth example)

First, a fifth example will be described. In the fifth example, a heartbeat may be fed back to the user U using a beating (pulsation) sound (for example, a sound such as "do, do, ..." as a sound corresponding to a heartbeat) rather than a numeral value. The correction information is displayed along with a beating sound. When the user U bodily feeds uneasy with the beating sound, an operation is performed on the correction information and the user U inputs a beating sound. The operation on the correction information includes at least one of an operation of inputting a sound based on time-series vital sensing data and an operation of performing tapping based on the time-series data vital sensing data. Beating may be presented to the user using not only a sound but also light, a video, or vibration, or a combination thereof.

Fig. 19 is a flowchart illustrating a flow of a process performed in response to an operation performed on correction information after the correction information is presented in this example. In this example, when an operation is performed on the correction information, a heart rate is corrected. Accordingly, the process illustrated in the flowchart of Fig. 19 is a specific process of the content of the process of steps ST1 to ST4 mainly described above.

After the process starts, in step ST511, the sensor 14 measures a heartbeat. Then, the process proceeds to step ST512.

In step ST512, a sound (beating sound) synchronized with the measured heartbeat is reproduced. As described above, the heartbeat may be presented to the user U using blinking, vibration, or a combination thereof in accordance with the heartbeat. Then, the process proceeds to step ST513.

In step ST513, the correction information is displayed. Fig. 20 is a diagram illustrating an example of the correction information in this example. In this example, the correction information is, for example, display in which an icon 51 resembling a microphone and an icon 52 resembling a tapping operation on the display unit 12 are arranged. The user U can input a beating sound which the user U feels in accordance with a favorite input method indicated by the icons 51 and 52. Then, the process proceeds to step ST514.

In step ST514, it is determined whether the correction information is tapped. When the correction information is not tapped, the process of step ST514 is repeated. When the correction information is tapped, the process proceeds to step ST515.

In step ST515, the beating which the user U feels at present is input by a sound or a tapping operation. For example, when the user U taps the icon 51 in the correction information, the user U makes a sound such as "do, do, ..." in accordance with the beating which the user U feels. When the user U taps the icon 52 in the correction information, the user U taps the display unit 12 in accordance with the beating which the user feels. Then, the process proceeds to step ST516.

In step ST516, a heart rate is calculated based on the input of the sound or the tapping operation by the user U and a measurement result is corrected with the calculated heart rate. The calculated heart rate is stored in the memory 16 or the like. A beating sound or the like based on the calculated heart rate may be reproduced. In this way, the user U can perform correction in real time when the user U feels uneasy with the measured beating sound.

### (Sixth example)

A sixth example is an example in which when the user U feels uneasy with a measured heartbeat, the heartbeat is corrected by measuring a heartbeat again in accordance with a different heartbeat measurement method and the corrected heartbeat is stored. In this example, in description, a heartbeat is measured in accordance with an optical measurement method in step ST1 of the overall flow.

Fig. 21 is a flowchart illustrating a flow of a process performed in response to an operation performed on correction information after the correction information is presented in this example. In this example, when the correction information is operated, a heart rate is corrected. Accordingly, the process illustrated in the flowchart of Fig. 21 is a specific process of the content of the process of steps ST2 to ST4 mainly described above.

After the process starts, in step ST611, the correction information is displayed on the display unit 12. The correction information in this example is information (for example, an icon) for correcting the heartbeat automatically or manually in real time without being limited to specific correction information. Accordingly, the correction information in this example is at least information with which it can be selected whether the heartbeat is corrected automatically or manually. As in the above-described example, the user U performs an operation on the correction information when the user U feels uneasy with a measurement result of the heartbeat. Then, the process proceeds to step ST612.

In step ST612, it is determined whether automatic correction of the operation performed on the correction information is selected. When the automatic correction is selected, the process proceeds to step ST613.

In step ST613, control is performed such that the band unit 3 is fastened. For example, the inside of the band unit 3 is filled with the air or a gas, and thus the thickness of the band unit 3 is considered to be thick. Then, the process proceeds to step ST614.

In step ST614, a heart rate is measured in accordance with a measurement method different from a heartbeat measurement method of an optical method. For example, the thickness of the band unit 3 is caused to be thick and a heart rate is measured in the same principle as that of a wrist type sphygmomanometer. Then, the process proceeds to step ST615.

In step ST615, a value of the heart rate measured in step ST614 is displayed on the display unit 12. The heart rate may be stored in the memory 16 or the like. The heart rate measured in step ST614 is applied to a measurement result and the measurement result is corrected. Then, the process ends.

When the operation on the correction information is the manual correction in the determination process of step ST612, the process proceeds to step ST616. In step ST616, the user U measures the heartbeat by himself or herself. For example, the user U measures the heart rate by palpation. Then, the process proceeds to step ST617.

In step ST617, the user U inputs the heart rate which is a measurement result to the wearable device 1. Then, the process proceeds to step ST618.

In step ST618, the heart rate input by the user U is displayed on the display unit 12. The heart rate may be stored in the memory 16 or the like. The heart rate input in step ST617 is applied to the measurement result to correct the measurement result. Then, the process ends.

According to this example, when the user U feels uneasy with the measurement result, the heartbeat can be measured in accordance with a more accurate method (a method of measuring the heartbeat directly by palpation, an oscillometric method, or the like) and the correction can be performed using the measured heartbeat.

### [Advantageous effects obtained in embodiment]

According to the above-described embodiment, for example, the following advantageous effects can be obtained.

In general, time-series vital sensing data is easily affected by noise and data may easily have low quality. Therefore, a user may feel uneasy with a measurement result of the time-series vital sensing data which has a tendency different from a subjective viewpoint of the user in some cases. However, according to the embodiment, by presenting the correction information, it is possible to correct highly accurate time-series vital sensing data based on a present subjective viewpoint of the user in real time. The correction result can be informed of by display or sound. The correction result can be stored. The correction result can be output to an external device to use healthcare.

According to the embodiment, from not only the software viewpoint but also the hardware viewpoint (for example, the position of the wearable device or the band length), it is possible to correct the setting for acquiring the time-series vital sensing data.

By using not only the display of the numerical value but also display, notification, or the like of an icon as the correction information, it is easy for the user to recognize the correction of the time-series vital sensing data.

For a person who does workout using a heartbeat, consumption calorie, or the like as a reference, an expected workout result cannot be obtained when an index serving as the reference is wrong. However, according to this example, since the time-series vital sensing data can be corrected to follow the bodily sensation in real time, it is possible to obtain a correct index appropriate for a situation of the user.

### <Modified examples>

The embodiment of the present disclosure has been described above specifically, but the content of the present disclosure is not limited to the above-described embodiment and various modifications can be made based on the technical spirit of the present disclosure. First, modified examples will be described.

In the above-described embodiment, while the time-series vital sensing data can be corrected in real time, there is concern of the user inappropriately performing the correction. Accordingly, countermeasures against the inappropriate correction may be taken.

For example, a log corrected by the user (a value before/after the correction) (an example of history information) remains on a memory to be transmitted to a predetermined server. The corrected log transmitted to the server is diagnosed by an expert such as a doctor and the doctor determines whether the corrected log is an appropriate log. Machine learning is performed to determine whether a corrected log is appropriate using the determination of the doctor as training data. The learning result is used later to automatically determine whether the corrected log is appropriate. When the corrected log is inappropriate, the user may be notified that the corrected log is appropriate. Based on the history information of the user, the time-series vital sensing data may be corrected.

A correctable range is set so that correction outside of the range may not be accepted by the wearable device. For example, in an example of a heartbeat, correction exceeding (40 to (220 - ages) bpm) may not be accepted. Reliability from an output value to a proper range may be calculated using a value output by the wearable device 1 as reliability of 100%. When the reliability is less than a threshold, the correction may not be accepted.

A specific example will be described with reference to Fig. 22. For example, it is assumed that an output value of a heart rate from the wearable device is set to 60 bpm and a user corrects the output value to 45 bpm. As illustrated in Fig. 22, a straight line is drawn for reliability from the output value to the proper range and a threshold of the reliability is set to 50%. Since 45 bpm is less than reliability of 50%, correction cannot be performed. In Fig. 22, an end point and an output value may be connected by a straight line or may be connected exponentially. In addition, when a distance at which the position of the wearable device can be corrected or an original estimated situation is "Exercise," a correctable range may be restricted, for example, in such a manner that correction to "Relaxation" is not permitted and only correction to "Tension/Excitement" is permitted.

First, other modified examples will be described. The time-series vital sensing data may be data related to other biological information (for example, data related to respiration rate) rather than data related to a heartbeat. Data related to a body temperature, VO2MAX, a blood pressure, blood sugar, or the like may be used. With regard to the data, it is difficult for the user to correct the time-series vital sensing data, but the user can change a setting for acquiring the time-series vital sensing data. When the user feels uneasy with a measurement result rather than correction, remeasurement may be performed. For example, a user interface (UI) in which a measured value and its likelihood (reliability) are simultaneously displayed and which can be operated when the user wants to perform remeasurement or recalculation is prepared. When the user feels uneasy with a measured value subjectively, the user may press a remeasurement button to remeasure calibrated time-series vital sensing data. The likelihood is calculated by estimating a situation of the user or the like at that time. Specifically, since there is a body motion of the user in exercise, the likelihood is lowered.

The above-described first to sixth process examples may be performed independently or may be performed in combination within a range in which there is no technical inconsistency.

The wearable device is not limited to a wristband type device and may be a device which can be worn on a neck, an ankle, a head, an ear, or the like. The information processing device according to the present disclosure may be a wearable device that is not a wearable device, but may be a device (for example, a stationary device located in a training facility). The information processing device according to the present disclosure may be a device integrated with another device (for example, wireless earphones).

The present disclosure can be realized by a device, a method, a program, a system, or the like. For example, by allowing a program that performs the functions described in the above-described embodiment to be downloadable and allowing a device that does not have the functions described in the above-described embodiments to download and install the program, it is possible to perform the control described in the embodiment in the device. The present disclosure can also be realized by a server that distributes the program. The factors described in the embodiments and the modified examples can be appropriately combined.

The content of the present disclosure is not construed to being limited by the advantageous effects exemplified in the present disclosure.

The present disclosure can be modified as follows.
(1) An information processing device including: a control unit configured to perform control such that information for correcting time-series vital sensing data acquired by a sensor is presented.
(2) The information processing device according to (1), wherein the information for the correction is information for correcting the time-series vital sensing data in real time.
(3) The information processing device according to (1) or (2), wherein the control unit performs control such that information indicating a tendency to change the time-series vital sensing data is presented as the information for the correction.
(4) The information processing device according to (3), wherein the information indicating the tendency to change the time-series vital sensing data includes at least two of an increase, a decrease, and a non-change.
(5) The information processing device according to (1), wherein the control unit performs control such that information indicating an estimated situation of a user with which a detection mode of the time-series vital sensing data is associated is presented as the information for the correction.
(6) The information processing device according to (5), wherein the information indicating the estimated situation of the user includes at least one of an exercise, relaxation, a tension, and excitement.
(7) The information processing device according to (1), wherein the control unit compares the time-series vital sensing data with a predetermined pattern and performs control such that the information for the correction is presented when a deviation between the time-series vital sensing data and the pattern is equal to or greater than a predetermined value as a comparison result.
(8) The information processing device according to (7), wherein the control unit performs control such that information for reporting that there is a section in which normal time-series vital sensing data is not acquired is presented as the information for the correction.
(9) The information processing device according to (1), wherein a setting for acquiring the time-series vital sensing data is changed in response to an operation on the information for the correction.
(10) The information processing device according to (9), wherein the setting is a setting related to a position of the sensor.
(11) The information processing device according to (10), wherein the setting is changed based on information regarding a feature of a user.
(12) The information processing device according to (1), wherein the time-series vital sensing data is corrected in response to an operation on the information for the correction.
(13) The information processing device according to (12), wherein a waveform corresponding to the time-series vital sensing data is corrected in response to an operation on the information for the correction.
(14) The information processing device according to (12) or (13), wherein a changeable range of the correction is restricted in accordance with reliability of the time-series vital sensing data.
(15) The information processing device according to (12), wherein the time-series vital sensing data is corrected based on history information of a user.
(16) The information processing device according to (12), wherein the operation on the information for the correction includes at least one of an operation of manually interpolating a deficient portion of the time-series vital sensing data, an operation of inputting a sound based on the time-series vital sensing data, and an operation of performing tapping based on the time-series vital sensing data.
(17) The information processing device according to (1), wherein a method of acquiring the time-series vital sensing data is changed in response to the operation on the information for the correction.
(18) The information processing device according to any one of (1) to (17), wherein the information processing device is configured as a wearable device.
(19) An information processing method including: performing control by a control unit such that information for correcting time-series vital sensing data acquired by a sensor is presented.
(20) A program causing a computer to perform an information processing method of performing control by a control unit such that information for correcting time-series vital sensing data acquired by a sensor is presented.

### [Reference Signs List]

- 1: Wearable device
- 2: Body unit
- 3: Band unit
- 11: Control unit
- 11a: Correction unit
- 12: Display unit
- 14: Sensor

## Claims

1. An information processing device comprising: a control unit configured to perform control such that information for correcting time-series vital sensing data acquired by a sensor is presented.

2. The information processing device according to claim 1, wherein the information for the correction is information for correcting the time-series vital sensing data in real time.

3. The information processing device according to claim 1, wherein the control unit performs control such that information indicating a tendency to change the time-series vital sensing data is presented as the information for the correction.

4. The information processing device according to claim 3, wherein the information indicating the tendency to change the time-series vital sensing data includes at least two of an increase, a decrease, and a non-change.

5. The information processing device according to claim 1, wherein the control unit performs control such that information indicating an estimated situation of a user with which a detection mode of the time-series vital sensing data is associated is presented as the information for the correction.

6. The information processing device according to claim 5, wherein the information indicating the estimated situation of the user includes at least one of an exercise, relaxation, a tension, and excitement.

7. The information processing device according to claim 1, wherein the control unit compares the time-series vital sensing data with a predetermined pattern and performs control such that the information for the correction is presented when a deviation between the time-series vital sensing data and the pattern is equal to or greater than a predetermined value as a comparison result.

8. The information processing device according to claim 7, wherein the control unit performs control such that information for reporting that there is a section in which normal time-series vital sensing data is not acquired is presented as the information for the correction.

9. The information processing device according to claim 1, wherein a setting for acquiring the time-series vital sensing data is changed in response to an operation on the information for the correction.

10. The information processing device according to claim 9, wherein the setting is a setting related to a position of the sensor.

11. The information processing device according to claim 10, wherein the setting is changed based on information regarding a feature of a user.

12. The information processing device according to claim 1, wherein the time-series vital sensing data is corrected in response to an operation on the information for the correction.

13. The information processing device according to claim 12, wherein a waveform corresponding to the time-series vital sensing data is corrected in response to an operation on the information for the correction.

14. The information processing device according to claim 12, wherein a changeable range of the correction is restricted in accordance with reliability of the time-series vital sensing data.

15. The information processing device according to claim 12, wherein the time-series vital sensing data is corrected based on history information of a user.

16. The information processing device according to claim 12, wherein the operation on the information for the correction includes at least one of an operation of manually interpolating a deficient portion of the time-series vital sensing data, an operation of inputting a sound based on the time-series vital sensing data, and an operation of performing tapping based on the time-series vital sensing data.

17. The information processing device according to claim 1, wherein a method of acquiring the time-series vital sensing data is changed in response to the operation on the information for the correction.

18. The information processing device according to claim 1, wherein the information processing device is configured as a wearable device.

19. An information processing method comprising: performing control by a control unit such that information for correcting time-series vital sensing data acquired by a sensor is presented.

20. A program causing a computer to perform an information processing method of performing control by a control unit such that information for correcting time-series vital sensing data acquired by a sensor is presented.
